# EUROPÄISCHE PATENTANMELDUNG

(11) **EP 1 070 499 A1**
(43) Veröffentlichungstag der Anmeldung: **24.01.2001**
(21) Anmeldenummer: 00109472.1
(22) Anmeldetag: 04.05.2000
(51) Int. Cl.: A61K 6/02, A61K 6/093

(54) **Verfahren zur physiologisch unbedenklichen Beschichtung von Kunststoffprothesen**

(30) Priorität: 21.07.1999 DE 19934225
(71) Anmelder: FRAUNHOFER-GESELLSCHAFT ZUR FÖRDERUNG DER ANGEWANDTEN FORSCHUNG E.V., 80636 München (DE)
(72) Erfinder: Rose, Klaus, Dr. Dipl.-Chem., 97318 Kitzingen (DE); Neumann, Gert, Dr. Dipl.-Chem, 13127 Berlin (DE); Borchmann, Michael, Dr., 49196 Bad Laer (DE); Möller, Bernhard, Dipl.-Ing., 97209 Veitshöchheim (DE)
(74) Vertreter: Pfenning, Meinig & Partner

(57) **Zusammenfassung**

Die Erfindung betrifft ein Verfahren zur physiologisch unbedenklichen Beschichtung von Kunststoffprothesen mit einem Lack auf der Basis von hydrolisierbaren Siliciumverbindungen, wobei der Lack aus zwei Komponenten besteht und die erste Komponente des Lackes eine ethylenisch ungesättigte Verbindung als nicht hydrolysierbare Gruppe aufweist und die zweite Komponente des Lackes über einen Merkaptorest als nichthydrolysierbare Gruppe verfügt und daß das Verhältnis von der ersten Komponente zur zweiten Komponente in bezug auf die ungesättigten Bindungen zu den Merkaptoresten im Bereich von 25:1 bis 1:1 liegt, und daß der Lack auf der Oberfläche der Kunststoffprothese aufgetragen und ausgehärtet wird.

## Beschreibung

Die Erfindung betrifft ein Verfahren zur physiologisch unbedenklichen Beschichtung von Kunststoffprothesen mit einem speziellen Lack auf Basis von hydrolysierbaren Si-Verbindungen.

Silanhydrolysatlacke zur Beschichtung von Zähnen und Dentalprothesen werden in der Patentschrift US 5,401,528 beschrieben. Die in dieser Schrift beschriebenen Systeme sind jedoch nicht hinsichtlich ihrer physiologischen Unbedenklichkeit charakterisiert. Insbesondere die von der Schrift beanspruchte Verwendung von Ge, Sn oder Al erscheint hinsichtlich der angestrebten Verwendung als besonders problematisch. Darüber hinaus ergibt sich noch Raum für Verbesserungen, insbesondere hinsichtlich der Barriereeigenschaften gegenüber den aus Zahnprothesen herausdiffundierenden Restmonomeren. Restmonomere kommen z.B. in kalthärtenden PMMA-Kunststoffen in der Größenordnung von 2 % bis 5 % vor. Diese Restmonomere können aufgrund ihrer Toxizität zu unangenehmen entzündlichen Erscheinungen der Mundschleimhaut, zu Geschmacksirritationen oder zum Brennen der Mundschleimhäute führen. Allergien können gegen alle Inhaltstoffe des Prothesenmaterials wie Monomere (MMA), Hydrochinon (Stabilisator), Dimethyl-p-toluidon (Katalysator), Benzoylperoxyd (Akzelerator) und Farbpigmente auftreten. Eine weitere verbesserungsfähige Eigenschaft der bekannten Beschichtungsmaterialien ist deren Aushärtungsregime. Die Aushärtung erfolgt in der Regel thermisch und/ oder durch Bestrahlung, wobei thermische bzw. photo-chemische Härtungskatalysatoren zugesetzt werden. Auch diese Katalysatoren erscheinen hinsichtlich der physiologischen Unbedenklichkeit als problematisch. Die angeführte thermische Aushärtung ist bei Dentalprothesen aufgrund der Stabilität des Prothesenkunststoffes nur unterhalb 100 °C möglich.

UV-härtende Prothesenlacke sind auf Basis von Methylmethacrylaten kommerziell auf dem Dentalmarkt erhältlich. Diese Lacke haben den Nachteil, daß sie monomeres MMA abgeben und keine Barriereeigenschaften gegenüber den Restmonomeren des Prothesenkunststoffes aufweisen. Die toxische und allergische Belastung der Mundschleimhaut wird durch diese Prothesenlacke eher erhöht als gemindert. Auch haben die Lacke auf PMMA-Basis die Eigenschaft zu vergilben, so daß die Prothesen nach kurzer Zeit unansehnlich werden.

In der Anmeldung EP 0 450 625 sind schnell aushärtende Silanhydrolysatlacke zur Herstellung kratzfester Beschichtungen bekannt. Diese Beschichtungssysteme lassen sich interessanterweise, entgegen US 5,401,528, bei Abwesenheit eines Photoinitiators innerhalb einer kurzen Aushärtungszeit aushärten. Die Anwendung einer Reihe von den in EP 0 450 625 beschriebenen Rezepturen als physiologisch unbedenklicher Lack zur Beschichtung von Dentalprothesen ist neu. Für eine Umsetzung in die Praxis erweist sich des weiteren die nicht ausreichende Lagerstabilität derartiger Prothesenlacke als wesentlicher Hinderungsgrund. In der Praxis wird dabei eine Gebrauchsdauer von mindestens 2 Jahren gefordert. Dabei gilt es schon als eine Einschränkung, wenn die Lagerung bei Temperaturen um 5 °C erfolgen muß.

Aufgabe der Erfindung ist es, einen nach der Aushärtung physiologisch unbedenklichen Lack zur Beschichtung von Prothesen bereitzustellen, wobei die resultierende Beschichtung eine hohe Kratzbeständigkeit und gute Haftung auf dem Substrat bei gleichzeitig ausgezeichneten Barriereeigenschaften, insbesondere gegenüber den aus Zahnprothesen herausdiffundierenden Restmonomeren, aufweisen soll. Weiterhin soll sich der Lack möglichst ohne Zugabe von Aktivatoren bzw. Initiatoren in möglichst kurzen Zeiten zufriedenstellend aushärten lassen. Des weiteren soll der Prothesenlack eine ausreichende Lagerstabilität aufweisen, um eine Umsetzung in die Praxis zu ermöglichen.

Die Fähigkeit Rauhigkeiten, die von der letzten Anpassung der Prothesen herrühren, einzuebnen und damit ein Polieren zu ersparen, ist eine weitere wesentliche Forderung an die Eigenschaften des Lackes.

Die Aufgabe wird durch die kennzeichnenden Merkmale des Anspruchs 1 gelöst. Die Unteransprüche zeigen vorteilhafte Weiterbildungen auf.

Erfindungsgemäß wird somit vorgeschlagen, Kunststoffprothesen mit einem Lack zu beschichten, der auf der Basis von hydrolysierbaren Silicium-Verbindungen der allgemeinen Formel (I)

SiXₙR₄₋ₙ

beruht. Wesentlich beim Anmeldungsgegenstand ist, daß 1 bis 40 Mol % aller an Silicium gebundenen Gruppen nicht hydrolysierbare Gruppen sind, wobei der Lack zwei Komponente enthält und die erste Komponente des Lackes dabei ethylenisch ungesättigte Verbindungen als nichthydrolysierbare Gruppen aufweist und die zweite Komponente des Lackes über einen Merkaptorest als nichthydrolysierbare Gruppe verfügt. Das Verhältnis von der ersten Komponente zur zweiten Komponenten in bezug auf die ungesättigten Bindungen zu den Merkaptoresten liegt erfindungsgemäß im Bereich von 25:1 bis 1:1. Der erfindungsgemäße Lack enthält entweder die zwei Komponenten als Mischung oder was bevorzugt ist, die beiden Komponenten werden getrennt gelagert und erst vor der Verarbeitung vermischt (Patentanspruch 6).

Wesentliches bei der zweiten Alternative ist die Möglichkeit der getrennten Hydrolyse der hydrolysierbaren Silicium-Verbindungen, so daß die ohne zusätzlichen Katalysator ablaufende organische Vernetzung zunächst durch die getrennte Lagerung der Komponenten nicht ablaufen kann.

Weiterhin ergibt sich die Möglichkeit einer Einstellung der Viskosität des Lackes durch Zugabe oligomerer linearer Siloxane, die gegebenenfalls auch in die Kondensationsreaktion einbezogen werden können. Mit der so erreichten erhöhten Viskosität ergibt sich die Möglichkeit, Rauhigkeiten, die von der letzten Anpassung der Prothesen herrühren, einzuebnen und damit ein Polieren zu ersparen.

Die Zugabe oligomerer linearere Polysiloxane, die in die Kondensationsreaktionen einbezogen werden, kann erfindungsgemäß zu jeder Zeit d.h. sowohl zu Beginn der Hydrolyse als auch nach Entfernung des Hydrolysekatalysators erfolgen. Besonders empfehlenswert ist die Zugabe nach erfolgter Hydrolyse, so daß die darin enthaltenen SiOH-Gruppen in die Kondensationsreaktion einbezogen werden. Sind in den oligomeren linearen Polysiloxane Phenylgruppen enthalten, ergibt sich ein zusätzlicher stabilisierender Effekt.

Besonders geeignete hydrolysierbare Siliciumverbindungen sind solche der o.a. allgemeinen Formel (I), in der die Reste X, die gleich oder verschieden sein können, insbesondere aus Alkoxygruppen, wie z. B. Methoxy, Ethoxy, n-Propoxy, 1-Propoxy und Butoxy und/oder Hydroxygruppen bestehen. Aber auch Halogen- (F, Cl, Br und J, insbesondere Cl und Br), Aryloxy- (z.B. Phenoxy), und/oder Acyloxygruppen, wie z.B. Acetoxy- und Propionyloxygruppen können X bilden, wenn die entstehenden Hydrolyseprodukte entfernt oder fest eingebunden werden. Die Reste R, die gleich oder verschieden sein können, sind aus Alkyl, wie z.B. Methyl, Ethyl, Propyl und Butyl, Alkenyl, wie z.B. Vinyl, 1-Propenyl, 2-Propenyl und Butenyl, Alkinyl wie Acetylenyl und Propargyl und Aryl, wie z.B. Phenyl und Naphthyl ausgewählt, wobei die soeben genannten Gruppen (mit Ausnahme von Halogen und Hydroxy) gegebenenfalls eine oder mehrere unter den Reaktionsbedingungen inerte Substituenten, wie z. B. Halogen und Alkoxy aufweisen können, und n eine ganze Zahl von 1 bis 4 ist. Die obigen Alkylreste schließen auch die entsprechenden cyclischen und arylsubstituierten Reste, wie z.B. Cyclohexyl und Benzyl ein, während die Alkenyl und Alkinylgruppen ebenfalls cyclisch sein können und die qenannten Arylgruppen auch Alkarylgruppen (wie Tolyl und Xylyl) mit einschließen sollen.

Die erfindungsgemäß vorhandenen Mercapto-(HS)-Reste befinden sich vorzugsweise an den obigen Alkyl- und Arylgruppen, insbesondere an den Alkylgruppen.

Neben den oben genannten besonders bevorzugten Resten X können als weitere, ebenfalls geeignete Gruppen, Wasserstoff und Alkoxyreste mit 5 bis 20, insbesondere 5 bis 10 Kohlenstoffatomen und Halogen- und Alkoxy-substituierte Alkoxygruppen (wie z.B. β-Methoxyethoxy) genannt werden. Weitere geeignete Gruppen R sind geradkettige, verzweigte oder cyclische Alkyl-, Alkenyl- und Alkinylreste mit 5 bis 20, insbesondere bis 10 Kohlenstoffatomen, wie z. B. n-Pentyl, n-Hexyl, Dodecyl und Octadecyl.

Da die Reste X im Endprodukt nicht vorhanden sind, sondern durch Hydrolyse verlorengehen, wobei das Hydrolyseprodukt früher oder später auch in irgendeiner geeigneten Weise entfernt werden muß, sind die Reste R besonders bevorzugt, die keine Substituenten tragen und zu Hydrolyseprodukten mit niedrigem Molekulargewicht, wie z. B. niederen Alkoholen, wie Methanol, Ethanol, Propanol, n-, i-, sek- und tert-Butanol führen.

Die Verbindungen der Formel (I) können ganz oder teilweise in Form von Vorkondensaten eingesetzt werden, d.h. Verbindungen, die durch teilweise Hydrolyse der Verbindungen der Formel (I) entweder allein oder im Gemisch mit anderen hydrolysierbaren Verbindungen, wie sie weiter unten näher beschrieben werden, entstanden sind. Derartige, im Reaktionsmedium vorzugsweise lösliche Oligomere können geradkettige oder cyclische, niedermolekulare Teilkondensate (Polyorganosiloxane) mit einem Kondensationsgrad von z.B. etwa 2 bis 100, insbesondere etwa 2 bis 6 sein. Unter den erfindungsgemäß vorhandenen nichthydrolysierbaren Gruppen R mit ethylenisch ungesättigter Doppelbindung, sind die obigen Alkenylreste mit 2 bis 4 Kohlenstoffatomen (insbesondere Vinyl) sowie (Meth)acryloxysubstituierte Alkyl- und Arylgruppen (insbesondere solche mit 2 bis 4 bzw. 6 bis 10 Kohlenstoffatomen, wie z. B. gamma-Methacryloxypropyl) und Styryl besonders bevorzugt. Nichthydrolysierbare Gruppen X mit Mercaptorest werden aus Mercaptoalkylresten mit 1 bis 6 Kohlenstoffatomen, wie z. B. 3-Mercaptopropyl, 4-Mercaptobutyl und 6-Mercaptohexyl, bevorzugt ausgewählt.

Die Herstellung des erfindungsgemäßen Lacks kann in auf diesem Gebiet üblicher Art und Weise erfolgen. Werden praktisch ausschließlich (bei der Hydrolyse relativ reaktionsträge) Siliciumverbindungen eingesetzt, kann die hydrolytische Kondensation in den meisten Fällen dadurch erfolgen, daß man den zu hydrolysierenden Siliciumverbindungen, die entweder als solche oder gelöst in einem geeigneten Lösungsmittel vorliegen, die stöchiometrisch erforderliche Menge Wasser bzw. gegebenenfalls einen Überschuß an Wasser bei Raumtemperatur oder unter leichter Kühlung direkt zugibt (vorzugsweise unter Rühren und in Anwesenheit eines Hydrolyse- und Kondensationskatalysators) und die resultierende Mischung daraufhin einige Zeit (ein bis mehrere Stunden) rührt. Unabhängig von der Reaktivität der anwesenden Verbindungen erfolgt die Hydrolyse in der Regel bei Temperaturen zwischen -20 °C und 130 °C, vorzugsweise zwischen 0 °C und 30 °C bzw. dem Siedepunkt des gegebenenfalls eingesetzten Lösungsmittels. Wie bereits angedeutet, hängt die beste Art und Weise der Zugabe von Wasser vor allem von der Reaktivität der eingesetzten Ausgangsverbindungen ab. So kann man z. B. die gelösten Ausgangsverbindungen langsam zu einem Überschuß an Wasser tropfen oder man gibt Wasser in einer Portion oder portionsweise den gegebenenfalls gelösten Ausgangsverbindungen zu. Es kann auch nützlich sein, das Wasser nicht als solches zuzugeben, sondern mit Hilfe von wasserhaltigen organischen- oder anorganischen Systemen in das Reaktionssystem einzutragen. Als besonders geeignet hat sich in vielen Fällen die Eintragung der Wassermengen in das Reaktionsgemisch mit Hilfe von feuchtigkeitsbeladenen Adsorbentien, z.B. Molekularsieben, und wasserhaltigen organischen Lösungsmitteln, z.B. 80 %igem Ethanol erwiesen.

Die Wasserzugabe kann auch über eine Reaktion erfolgen, bei der Wasser gebildet wird, z.B. bei der Esterbildung aus.Säure und Alkohol. Wenn ein Lösungsmittel verwendet wird, kommen neben den niederen aliphatischen Alkoholen (z.B. Ethanol und Isopropanol) auch Ketone, vorzugsweise niedere Dialkylketone, wie Aceton und Methylisobutylketon, Ether, vorzugsweise niedere Dialkylether wie Diethylether und Dibutylether, THF, Amide, Ester, insbesondere Essigsäureethylester, Dimethyiformamid und deren Gemische in Frage. Werden nur hydrolysierbare Siliciumverbindungen eingesetzt, kann sich der Einsatz von niederen Dialkylethern als Lösungsmittel als besonders vorteilhaft erweisen. Insbesondere wirkt die Verwendung dieser Ester einer zu schnellen Gelierung des Lacks entgegen, wenn dieser relativ viele Mercaptoreste aufweist.

Erfindungsgemäß bevorzugt eingesetzte Hydrolyse- und Kondensationskatalysatoren sind protonenabspaltende Verbindungen. Beispiele hierfür sind organische- und anorganische Säuren, wie Salzsäure, Essigsäure, Ameisensäure und/oder Zitronensäure, wobei Salzsäure als Katalysator besonders bevorzugt wird. Die Gesamtkatalysatorkonzentration kann z.B. bis zu 3 Mol pro Liter betragen. Wesentlich für das Erreichen der physiologischen Unbedenklichkeit ist die gute Löslichkeit in Wasser, die es ermöglicht, nach Erreichen einer mittleren Kondensationsstufe den Katalysator mit Wasser auszuwaschen.

Die Ausgangsverbindungen müssen nicht notwendigerweise bereits alle zu Beginn der Hydrolyse (Polykondensation) vorhanden sein, sondern in bestimmten Fällen kann es sich sogar als vorteilhaft erweisen, wenn nur ein Teil dieser Verbindungen zunächst mit Wasser in Kontakt gebracht wird und später die restlichen Verbindungen zugegeben werden. Für die erfindungsgemäß hergestellten lagerstabilen Komponenten ist die Hydrolyse wesentlicher Teil des erfindungsgemäßen Vorgehens.

Der erfindungsgemäße Lack wird deshalb bevorzugt hergestellt durch hydrolytische Kondensation einer oder mehrerer hydrolysierbarer Siliciumverbindungen wie vorstehend beschrieben, sowie gegebenenfalls einer oder mehrerer hydrolysierbarer Verbindungen von Zirkonium oder Aluminium in einer Menge von höchstens 30 Molprozent, bezogen auf die Gesamtmenge an Silicium und Zirkoniumverbindungen, wobei 1 bis 40 Molprozent aller an die obigen Elemente gebundenen Gruppen nichthydrolysierbare Gruppen sind, die eine ethylenisch ungesättigte Bindung aufweisen und zusätzlich an den obigen Elementen nichthydrolysierbare Gruppen, die über einen Mercaptorest verfügen, in einer solchen Menge vorhanden sind, daß das Verhältnis von ethylenisch ungesättigten Bindungen zu Mercaptoresten in bzw. an nichthydrolysierbaren Gruppen 25:1 bis 1:1 beträgt.

Um insbesondere bei Verwendung von verschiedenen hydrolysierbaren Siliciumverbindungen Ausfällungen während der Hydrolyse und Polykondensation so weit wie möglich zu vermeiden, wird es in diesem Fall bevorzugt, die Wasserzugabe in mehreren Stufen, z.B. in drei Stufen, durchzuführen. Dabei wird in der ersten Stufe z.B. ein Zehntel bis ein Zwanzigstel der zur Hydrolyse stöchiometrisch benötigten Wassermenge zugegeben. Nach kurzem Rühren folgt die Zugabe von einem Fünftel bis zu einem Zehntel der stöchiometrischen Wassermenge und nach weiterem kurzen Rühren wird schließlich eine stöchiometrische Wassermenge zugegeben, so daß am Schluß ein leichter Wasserüberschuß vorliegt. Die Kondensationszeit richtet sich nach Art und Mengenanteil der jeweiligen Ausgangskomponenten, dem gegebenenfalls verwendeten Katalysator, der Reaktionstemperatur etc. Im Allgemeinen erfolgt die Polykondensation bei Normaldruck, sie kann jedoch auch bei einem erhöhten oder verringerten Druck durchgeführt werden.

Der erfindungsgemäße Lack kann zur Versiegelung von sämtlichen Prothesenkunststoffen, speziell den warm- und kalthärtenden Polymethylmethacrylaten von kieferorthopädischen Kunststoffen, Schienenkunststoffen, Verblendkunststoffen (Kompositen) für festsetzenden Zahnersatz, für Kompositfüllungen, Compomerfüllungen, Zementfüllungen und sonstigen Füllungsmaterialien verwendet werden. Auch ist eine Beschichtung von individuell und fabrikmäßig hergestellten Hörgeräten und den dafür verwendeten Kunststoffen, welche Kontakt mit der Ohrmuschel und dem Gehörgang haben, möglich.

Für die Beschichtung von Prothesenkunststoffen wird der durch Vermischen der Einzelkomponenten erhaltene Lack entweder als solcher oder nach teilweiser oder nahezu vollständiger Entfernung des verwendeten Lösungsmittels bzw. des während der Reaktion gebildeten Lösungsmittels im erfindungsgemäßen Beschichtungsverfahren eingesetzt. In einigen Fällen kann es sich als vorteilhaft erweisen, in dem nach der Polykondensation erhaltenen Lack das überschüssige Wasser und das gebildete und gegebenenfalls zusätzlich eingesetzte Lösungsmittel durch ein anderes Lösungsmittel zu ersetzen, um den Lack zu stabilisieren. Zu diesem Zweck kann der Lack z.B. im Vakuum bei leicht erhöhter Temperatur (bis maximal 80 °C) so weit eingedickt werden, daß er noch problemlos mit einem anderen Lösungsmittel aufgenommen werden kann. Als Ersatz-Lösungsmittel haben sich insbesondere Essigester und Toluol bewährt. Derartig stabilisierte Lacke sind dann ohne optische Änderung und ohne merkliche Viskositätszunahme über mehrere Wochen stabil.

Für eine Aushärtung des Lacks nach dem Auftrag auf die Prothese hat sich die Bestrahlung mit UV-Licht bewährt. Überraschenderweise wurde festgestellt, daß für die UV-Härtung des erfindungsgemäß hergestellten Lacks die Anwesenheit eines Photoinitiators nicht erforderlich ist. Erfindungsgemäß wird der wie oben beschrieben hergestellte Lack auf Dentalprothesen aus allen denkbaren Kunststoffen aufgebracht werden. Um eine ausgezeichnete Haftung des Überzugs auf dem Kunststoffsubstrat zu gewährleisten, empfiehlt es sich in der Regel, das Kunststoffsubstrat vor der Beschichtung einer Oberflächenbehandlung, z.B. durch Auslaugen und Grundieren, mit einem Primer, Coronabehandlung usw., zu unterziehen. Überraschenderweise wurde festgestellt, daß eine derartige Oberflächenbehandlung im Falle des Polymethylmethacrylat auch weggelassen werden kann und daß bei einer Vorbehandlung der zu beschichtenden Oberfläche entsprechend der im zahntechnischen Labor geübten Praxis, eine ausgezeichnete Haftung zwischen Substrat und Überzug erzielt wird. Vor der Härtung wird das Lösemittel aus dem aufgetragenen Lack vorzugsweise durch Verdunstung entfernt. Danach kann durch Bestrahlen (z.B. mit einem UV-Strahler, einem Laser usw.) in an sich bekannter Weise die Härtung durchgeführt werden. Bei der Beschichtung von Prothesen kann es sich von Vorteil erweisen, nach der Strahlungshärtung eine thermische Nachhärtung mittels heißen Wassers durchzuführen, insbesondere um einen eventuell vorhandenen Überschuß an ungesättigten Gruppen oder noch vorhandenes Lösungsmittel zu entfernen.

Die UV-Härtung läßt sich je nach Leistung des Strahlers in weniger als 60 Sekunden durchführen.

Für die Aushärtung mit normalem oder sichtbarem Licht erweist sich die Zugabe eines Starters, wie Campherchinon als unumgänglich. Die Bestrahlung erfolgt bevorzugt mit Licht einer Wellenlänge von 350 bis 500 nm. Zusätzlich zu diesem Starter können tertiäre Amine als Akzeleratoren erfindungsgemäß eingesetzt werden. Auch hat es sich als vorteilhaft erwiesen, Beschleuniger wie Dimethylaminopropylsilan zuzusetzen. Überraschenderweise beeinträchtigen Zugaben von weniger als 1 % der Aktivatoren nicht die physiologische Unbedenklichkeit.

Mit dem erfindungsgemäßen Verfahren werden in der Regel Schichtdicken von 5 bis 50 µm, insbesondere 10 bis 20 µm erreicht. Selbstverständlich ist das erfindungsgemäße Verfahren nicht darauf beschränkt, nur eine einzige Lackschicht auf dem Substrat aufzubringen, sondern es besteht auch die Möglichkeit, nach dem Auftragen und gegebenenfalls Aushärten einer Schicht weitere Schichten aufzutragen und damit zu Multi-Layer-Strukturen zu gelangen.

Das erfindungsgemäße Beschichtungsverfahren bzw. der erfindungsgemäße Lack führt insbesondere zu den folgenden überraschenden Vorteilen:

Es können hoch kratzfeste, gut auf dem Kunststoffsubstrat haftende Überzüge erhalten werden, die eine sehr gute Barrierewirkung zeigen.

Besonders bei den thermisch wenig belastbaren Substraten kann durch UV-Behandlung bei milden Bedingungen eine gute Aushärtung in kürzester Zeit erzielt werden.

Die UV-Härtung läßt sich je nach Leistung des Strahlers in weniger als 60 Sekunden durchführen. Dabei ist die Tatsache, daß bei der UV-Härtung auf einen Photoinitiator verzichtet werden kann, wesentlich für die vorliegenden Erfindung.

Im Vergleich zu Systemen auf Basis von Silanen mit ethylenisch ungesättigter Doppelbindung im Molekül, wird durch den erfindungsgemäßen Zusatz von hydrolysierbaren Verbindungen mit Mercaptogruppen sowohl die Endhärte als auch die Reaktionsgeschwindigkeit erheblich verbessert.

Im Falle von Polymethylmethacrylat als bewährtem Prothesenkunststoff, kann eine ausgezeichnete Haftung ohne Vorbehandlung der Oberfläche des Substrats erzielt werden.

Die folgenden Beispiele erläutern die vorliegende Erfindung, ohne sie zu beschränken:

### Beispiel 1

Es werden jeweils eine Menge von 0,5 Mol Vinyltrimethoxysilan und 0,5 Mol Mercaptopropyltriethoxysilan bei 15 bis 20 °C mit 1,5 Mol Wasser, das in Form von verdünnter Salzsäure (0,1-normal) eingesetzt wird, versetzt. Danach werden die Reaktionsmischungen unter leichter Kühlung (2 Stunden lang) bei der angegebenen Temperatur gerührt. Daraufhin wurden jeweils 125 ml (250 ml pro Mol Alkoxysilan) Ethylacetat zugesetzt und zunächst pro Mol Alkoxysilan bei 30 bis 35 °C ca. 140 g Lösungsmittelgemisch abgezogen. Die danach zähflüssige Lösung wurde nochmals in der gleichen Menge Ethylacetat aufgenommen und die erhaltenen Lösungen zweimal bis zur Chloridfreiheit (mit Silbernitrat geprüft) mit Wasser gewaschen und auf die gewünschte Konzentration erneut einrotiert.

Die kinematische Viskosität der so erhaltenen Teilkomponenten des Lacks lag bei etwa 5 mm²/Sekunde und der Feststoffgehalt betrug jeweils 35 Gewichtsprozent. Getrennt bei Raumtemperatur gelagert, erhöht sich die Viskosität der Komponenten innerhalb eines halben Jahres um weniger als 1 %. Zur Verarbeitung werden beide Komponenten 1:1 zu einem anwendungsbereiten Lack vermischt.

Dieser Lack läßt sich nach 14-tägiger Lagerung noch problemlos verarbeiten. Der wie oben hergestellte Lack wurde mit einem Pinsel dann auf ein Substrat aus Polymethylmethacrylat aufgetragen. Bei der Beschichtung im Tauchverfahren wurde eine Schichtdicke von 5 bis 10 µm erzielt.

Der aufgetragene und bei Raumtemperatur angetrocknete Lack wurde dann unter den folgenden Bedingungen gehärtet:
UV-Härtung, 500 W, 120 Sekunden, Abstand 17 cm (Loctite-Gerät)
   oder
5 m/Minute Bandgeschwindigkeit bei 2 x 2000 W (Beltron-Gerät)

Der so gehärtete Lack wurde hinsichtlich seiner Eigenschaften untersucht und es wurden die folgenden Ergebnisse erhalten:
Ritzhärte 10 g
Bleistifthärte 5H
Abriebtest nach 100 bzw. 300 Zyklen 2 bzw. 8 %

Bemerkenswert an diesen Ergebnissen ist unter anderem, daß diese Werte nach einer vierwöchigen Schwitzwasserbelastung unverändert blieben.

### Beispiel 2

Es werden eine Menge von 0,6 Mol Vinyltrimethoxysilan und 0,4 Mol Mercaptopropyltriethoxysilan bei 15 bis 20 °C mit 1,5 Mol Wasser, das in Form von verdünnter Salzsäure (0,1-normal) eingesetzt wird, versetzt. Danach wird die Reaktionsmischung unter leichter Kühlung (2 Stunden lang) bei der angegebenen Temperatur gerührt. Daraufhin wurden 30 g eines oligomeren Polysiloxan (Dow Corning intermediate Z 6018) in 250 ml Ethylacetat gelöst und dem Hydrolyseansatz zugesetzt und zunächst bei 40 bis 50 °C ca. 140 g Lösungsmittelgemisch langsam abgezogen. Die danach zähflüssige Lösung wurde nochmals in der gleichen Menge Ethylacetat aufgenommen und der erhaltene Lack zweimal bis zur Chloridfreiheit (mit Silbernitrat geprüft) mit Wasser gewaschen und auf die gewünschte Konzentration erneut einrotiert.

Die kinematische Viskosität der so erhaltenen Teilkomponenten des Lacks lag bei etwa 5,8 mm²/Sekunde und der Feststoffgehalt betrug 35 Gewichtsprozent. Bei Kühlschranktemperatur gelagert, erhöht sich die Viskosität der Komponenten innerhalb eines halben Jahres um weniger als 1 %.

Dieser Lack läßt sich nach 14tägiger Lagerung noch problemlos verarbeiten. Der wie oben hergestellte Lack wurde mit einem Pinsel dann auf ein Substrat aus Polymethylmethacrylat aufgetragen. Der aufgetragene und bei Raumtemperatur angetrocknete Lack wurde dann unter den folgenden Bedingungen gehärtet:
UV-Härtung, 500 W, 120 Sekunden, Abstand 17 cm (Loctite-Gerät)
oder 5m/Minute Bandgeschwindigkeit bei 2x2000 W (Beltron-Gerät)

### Beispiel 3

Es werden eine Menge von 0,6 Mol Vinyltrimethoxysilan und 0,4 Mol Mercaptopropyltriethoxysilan bei 15 bis 20 °C mit 1,5 Mol Wasser, das in Form von verdünnter Salzsäure (0,1-normal) eingesetzt wird, versetzt. Danach wird die Reaktionsmischung unter leichter Kühlung (2 Stunden lang) bei der angegebenen Temperatur gerührt. Daraufhin wurden 30 g eines oligomeren Polysiloxan (Dow Corning intermediate Z 6018) in 250 ml Ethylacetat gelöst und dem Hydrolyseansatz zugesetzt und zunächst bei 40 bis 50 °C ca. 140 g Lösungsmittelgemisch langsam abgezogen. Die danach zähflüssige Lösung wurde nochmals in der gleichen Menge Ethylacetat aufgenommen und der erhaltene Lack zweimal bis zur Chloridfreiheit (mit Silbernitrat geprüft) mit Wasser gewaschen. Dann werden zu dem Lackansatz (Feststoffgehalt etwa 18 %) 1 % Dimethylaminopropylsilan zugegeben und der Ansatz nach kurzem Rühren auf die gewünschte Konzentration erneut einrotiert.

Die kinematische Viskosität der so erhaltenen Teilkomponenten des Lacks lag bei etwa 7,9 mm²/Sekunde und der Feststoffgehalt betrug 35 Gewichtsprozent. Bei Kühlschranktemperatur gelagert, erhöht sich die Viskosität der Komponenten innerhalb eines halben Jahres um weniger als 1 %. Zur Verarbeitung werden dem anwendungsbereiten Lack 0,6 % Campherchinon zugemischt. Der mit einem Pinsel auf PMMA-Scheiben aufgetragene und bei Raumtemperatur angetrocknete Lack wurde dann mit einem handelsüblichen Lichtaushärtungsgerät (Luxomat RSB) ausgehärtet.

### Beispiel 4

30 Scheiben aus kommerziellen Polymethylmethacrylatkaltpolymerisat wurden gemäß Beispiel 1 mit dem erfindungsgemäßen Lack getaucht und ausgehärtet. Anschließend wurde an diesen Proben die Prüfung der Biokompatibilität des Dentalwerkstoffs durch Untersuchung der Verträglichkeit mit Zellkulturen vorgenommen. Dazu wurde in Anlehnung an ISO 10993-5 der Grad der Beeinflussung des Zellwachstums durch lösliche Bestandteile aus dem Kunststoffmaterial bzw. aus dem Beschichtungswerkstoff ermittelt.

Jeweils 8 Prüfkörper mit und ohne Beschichtung wurden 72 Stunden bei 37 °C im Zellkulturmedium gelagert (3,6 cm²/ml Medium), um ein mit Löslichkeitsprodukten (z.B. monomere Bestandteile) kontaminiertes Eluat herzustellen. Dieses Eluat wurde dann in verschiedenen Konzentrationen (2,5 %; 25 %; 50 %; 75 %) dem Zellkulturmedium hinzugefügt. Anschließend wurde die Zellaktivität von Mausfibroblasten nach 72 Stunden mit dem MTT-Test ermittelt.

Bei unbeschichteten Proben ergibt dieser Test eine 37 %ige Wachstumshemmung (gering zytotoxisch) bei Zusatz von 75 % Eluat, bei beschichteten Proben liegt die Wachstumshemmung bei gleicher Eluatkonzentration bei 0 %. Hierdurch wird die ausgezeichnete Barrierewirkung der Beschichtung eindrucksvoll deutlich. Auch der Lack selbst ist als biokompatibel anzusehen, gleichzeitig verhindert er die Diffusion von monomeren Bestandteilen des Grundwerkstoffes. Durch dieses Versiegelungsmaterial kann die Verträglichkeit von Prothesenkunststoffen und weiterer zahnärztlicher Materialien hinsichtlich der Toxizität und Allergenität wesentlich verbessert werden.

## Patentansprüche

1. Verfahren zur physiologisch unbedenklichen Beschichtung von Kunststoffprothesen mit einem Lack auf der Basis von hydrolisierbaren Siliciumverbindungen, wobei die Siliciumverbindungen durch die allgemeine Formel I definiert sind
SiXₙR₄₋ₙ (I)
worin der Rest X der gleich oder verschieden sein kann, ausgewählt ist aus Halogen, Alkoxy, Aryloxy, Acyloxy und Hydroxy und der Rest R der gleich oder verschieden sein kann, ausgewählt ist aus Alkyl, Alkenyl, Alkinyl und Aryl, und daß die oben genannten Gruppen gegebenenfalls eine oder mehrere unter den Reaktionsbedingungen inerte Substituenten tragen können und n eine ganze Zahl von 1 bis 4 ist mit der Maßgabe, daß 1 bis 40 Mol-% aller an Silicium gebundenen Gruppen nichthydrolysierbare Gruppen sind, wobei der Lack aus zwei Komponenten besteht und die erste Komponente des Lackes eine ethylenisch ungesättigte Verbindung als nicht hydrolysierbare Gruppe aufweist und die zweite Komponente des Lackes über einen Merkaptorest als nichthydrolysierbare Gruppe verfügt und daß das Verhältnis von der ersten Komponente zur zweiten Komponente in bezug auf die ungesättigten Bindungen zu den Merkaptoresten im Bereich von 25:1 bis 1:1 liegt, und daß
der Lack auf der Oberfläche der Kunststoffprothese aufgetragen und ausgehärtet wird.

2. Verfahren nach Anspruch 1, dadurch gekennzeichnet, daß die hydrolysierbaren Verbindungen der allgemeinen Formel I der ersten Komponente und/oder der zweiten Komponente ganz oder teilweise in Form von Vorkondensaten vorliegen.

3. Verfahren nach Anspruch 1, dadurch gekennzeichnet, daß die hydrolysierbaren Verbindungen der allgemeinen Formel I der ersten Komponente und/oder der zweiten Komponente in vollständig hydrolysierter Form vorliegen.

4. Verfahren nach mindestens einem der Ansprüche 1 bis 3, dadurch gekennzeichnet daß die nichthydrolysierbaren Gruppen mit ethylenisch ungesättigten Doppelbindungen ausgewählt sind aus Alkenyl, insbesondere Vinyl, 1-Propenyl, 2-Propenyl und Butenyl, Styryl, (Meth) acryloxyalkyl und Mischungen hiervon.

5. Verfahren nach einem der Ansprüche 1 bis 4, dadurch gekennzeichnet, daß die nichthydrolysierbaren Gruppen mit Mercaptoresten ausgewählt sind aus Mercaptoalkylresten, insbesondere 3-Mercaptopropyl, 4-Mercaptobutyl, 6-Mercaptohexyl und Mischungen hiervon.

6. Verfahren nach mindestens einem der Ansprüche 1 bis 5, dadurch gekennzeichnet, daß die beiden Komponenten des Lacks getrennt gelagert und erst vor der Anwendung vermischt werden.

7. Verfahren nach mindestens einem der Ansprüche 1 bis 6, dadurch gekennzeichnet, daß die Aushärtung des Lacks nach dem Auftrag durch Bestrahlung mit UV-Licht erfolgt.

8. Verfahren nach mindestens einem der Ansprüche 1 bis 7 dadurch gekennzeichnet, daß nach der Hydrolyse der hydrolysierbaren Verbindungen der Formel I der ersten Komponente und/oder der zweiten Komponente ein oligomeres Polysiloxan zugesetzt wird.

9. Verfahren nach Anspruch 8, dadurch gekennzeichnet, daß dem Lack ein Starter, bevorzugt Campherchinon zugegeben wird.

10. Verfahren nach mindestens einem der Ansprüche 1 bis 9, dadurch gekennzeichnet, daß dem Lack ein Beschleuniger, bevorzugt Dimethylaminopropylsilan zugegeben wird.

11. Verfahren nach mindestens einem der Ansprüche 1 bis 10, dadurch gekennzeichnet, daß Starter und/oder Beschleuniger der Merkaptogruppen enthaltenen Komponenten zugesetzt werden.

12. Verfahren nach mindestens einem der Ansprüche 1 bis 11, dadurch gekennzeichnet, daß die Aushärtung des Lacks durch Bestrahlung mit normalem bzw. sichtbarem Licht, bevorzugt von 350 bis 500 nm erfolgt.

13. Verfahren nach mindestens einem der Ansprüche 1 bis 12, dadurch gekennzeichnet, daß eine Schichtdicke im Bereich von 5 bis 50 µm, besonders von 10 bis 20 µm aufgebracht wird.

14. Verfahren nach mindestens einem der Ansprüche 1 bis 13, dadurch gekennzeichnet, daß zwei bis zehn Schichten mit einer Schichtdicke im Bereich von 5 bis 50 µm übereinander aufgebracht werden.

15. Verfahren nach mindestens einem der Ansprüche 1 bis 14, dadurch gekennzeichnet, daß der Lack auf Polymethylmetacrylat (PMMA) aufgebracht wird.
